# EUROPEAN PATENT SPECIFICATION

(11) **EP 1 369 695 B1**
(45) Date of publication and mention of the grant of the patent: **15.03.2006**
(21) Application number: 02011964.0
(22) Date of filing: 29.05.2002
(51) Int. Cl.: G01N 33/68, C07K 14/705

(54) **Method for identifying type-I interferon responsive MS patients by determining TRAIL expression**
Verfahren zur Identifizierung von auf Ifn-Beta ansprechenden multiple Sklerose Patienten, durch die Bestimmung der Expression von Trail
Identification de patients souffrant d'une sclérose en plaques qui est susceptible de répondre à un traitement par IFN-beta, par la détermination de l'expression de trail

(43) Date of publication of application: 10.12.2003
(73) Proprietor: Charité - Universitätsmedizin Berlin, 10117 Berlin (DE)
(72) Inventor: WANDINGER, Klaus-Peter, 13187 Berlin (DE); LÜNEMANN, Jan, 10115 Berlin (DE); ZIPP, Frauke, 13501 Berlin (DE)
(74) Representative: Goddar, Heinz J.

(56) References cited:
- WO-A-97/33899
- FR-A- 2 766 713
- WANDINGER KLAUS-PETER ET AL: "Complex immunomodulatory effects of interferon-beta in multiple sclerosis include the upregulation of T helper 1-associated marker genes." ANNALS OF NEUROLOGY, vol. 50, no. 3, September 2001 (2001-09), pages 349-357, XP008010479 ISSN: 0364-5134
- WOSIK KAROLINA ET AL: "Interferon b (IFNb) modulates death receptor DR4 and DR5 expression on oligodendrocytes and their ligand TRAIL on T cells." NEUROLOGY, vol. 56, no. 8 Supplement 3, 24 April 2001 (2001-04-24), page A152 XP008010460 53rd Annual Meeting of the American Academy of Neurology;Philadelphia, PA, USA; May 05-11, 2001 ISSN: 0028-3878
- WANDINGER KLAUS-PETER ET AL: "Diminished production of type-I interferons and interleukin-2 in patients with multiple sclerosis." JOURNAL OF THE NEUROLOGICAL SCIENCES, vol. 149, no. 1, 1997, pages 87-93, XP002220790 ISSN: 0022-510X
- JEFFERY DOUGLAS R: "Relationship between disease activity and dose-response relationships with beta interferon therapies in the treatment of multiple sclerosis." JOURNAL OF THE NEUROLOGICAL SCIENCES, vol. 178, no. 1, 1 September 2000 (2000-09-01), pages 2-9, XP002220791 ISSN: 0022-510X

## Description

This invention relates to a method for identifying MS patients susceptible to type I-Interferon (IFN), in particular IFN-β, and for monitoring IFN therapy in MS patients by determining TRAIL gene and/or protein expression as a marker.

Multiple Sclerosis (MS) affects approximately 1 million individuals worldwide and is the most common disease of the central nervous system (CNS) that causes prolonged and severe disability in young adults. Although its etiology remains elusive, strong evidence supports the concept that a T cell-mediated inflammatory process against self-molecules within the white matter of the brain and spinal cord underlies its pathogenesis (Martin *et al*. 1992, *Annu Rev Immunol* **10**: 153-187). Since myelin-reactive T cells are present in both MS patients and healthy individuals (Pette *et al*. 1990, *Neurology* **40**: 1770-1776; Hohlfeld *et al.* 1995, *Neurology* **45** (suppl 6): S33-S38), the primary immune abnormality in MS most likely involves failed regulatory mechanisms that lead to an enhanced T cell activation status and less stringent activation requirements (Zang *et al.* 1994, *J Exp Med* **179**: 973-984; Markovic-Plese *et al.* 2001, *J Clin Invest* **108**: 1185-1194).

Interferon-beta (IFN-β), a type-I Interferon (IFN), is a pleiotropic cytokine with immunomodulatory properties and has become a global standard in the treatment of MS. Despite the well documented efficacy in responders to this medication, a substantial number of patients fail to respond to IFN-β. Why IFN-β therapy is or is not effective with respect to MS, and how IFN-β alters the clinical course of MS remains unclear (The IFNB Multiple Sclerosis Study Group and the University of British Columbia MS/MRI Analysis Group 1995, *Neurology* **45**: 1277-1285; Jacobs *et al.* 1996, *Ann Neurol* **39**: 285-294; PRISMS Study Group 1998, *Lancet* **352**: 1498-1504). Putative mechanisms of action include the inhibition of T cell proliferation, regulation of a large number of cytokines, and blocking of blood-brain barrier opening via interference with cell adhesion, migration and matrix metalloproteinase activity (Rudick *et al*. 1996, *Ann Neurol* **40**: 618-627; Wang *et al*. 2000, *J Immunol* **165**: 548-557; Stone *et al*. 2000, *Ann Neurol* **37**: 611-619). Understanding how IFN-β works in MS, however, is imperative for a development of markers that would allow predicting an individual's chance of a positive IFN-β response. In the face of the costs of IFN-β therapy as well as possible side-effects for the patient, such a prediction might be valuable in increasing the efficacy of current and future strategies to treat patients suffering from MS.

Consequently, there is a need for identifying markers which correlate with the clinical outcome of IFN-β therapy in MS patients, and which are usable in a method for predicting therapy response of an individual patient.

Recently, a number of genes involved in innate and specific immune responses that are potential effector targets of IFN-β in MS were identified (Wandinger *et al.* 2001, *Ann-Neurol* **50:** 349-357). Tumor necrosis factor (TNF)-related apoptosis inducing ligand (TRAIL; also referred to as APO2L), a novel member of the TNF/nerve growth factor superfamily (Wiley *et al*. 1995, *Immunity* **3**: 673-682), was among the genes that were consistently up-regulated by IFN-β *in vitro.* Interestingly, TRAIL has also been shown to mediate the induction of endogenous IFN-β, thereby amplifying the effects of this therapy (Kumar-Sinha *et al*. 2002, *J Biol Chem* **277**: 575-585).

Apart from its apoptosis inducing effects (Wiley *et al*. 1995, *Immunity* **3**: 673-682); TRAIL was recently shown to exert potent anti-inflammatory properties at the T cell level (Song *et al.* 2000, *J Exp Med* **191**: 1095-1104; Hilliard *et al.* 2001, *J Immunol* **166**: 1314- 1319; Lünemann *et al*. 2002, *J Immunol* **168:** 4881-4888). Studies on animal models of autoimmune diseases demonstrated that systemic neutralization by TRAIL receptor 2 led to exacerbation of collagen-induced arthritis and experimental autoimmune encephalomyelitis and suggested an influence of TRAIL on T cell growth and effector function (Song *et al.* 2000, *J Exp Med* **191:** 1095-1104; Hilliard *et al.* 2001, *J Immunol* **166**: 1314-1319). In the human immune system, Lünemann *et al*. (2002, *J Immunol* **168:** 4881-4888) have very recently shown that TRAIL fails to induce apoptosis, but inhibits the activation of autoreactive and foreign antigen-specific T cells. In particular, TRAIL negatively regulates calcium influx through store-operated calcium release-activated calcium channels which is crucial to lymphocyte activation, and moreover inhibits subsequent cell cycle progression as well as cytokine production independently of the T cell's antigen-specificity or the T helper phenotype (Lünemann *et al*. 2002 *J. Immunol* **168**: 4881-4888). This impact of TRAIL on human T cell effector functions suggests a role of the TRAIL system in the pathogenesis of MS in which activated myelin-specific T cells are thought to mediate the inflammatory brain damage. This notion is supported by the fact that TRAIL was found to be elevated in peripheral immune cells of MS patients during the natural disease course, and that levels were highest in patients with relapsing-remitting MS at the time of clinical remission (Huang *et al.* 2000, *Neurology* **55**: 928-934).

In addition to its immunoregulatory properties on human T cells, TRAIL was shown to induce apoptotic cell death in nontransformed human hepatocytes and brain cells (Jo *et al* 2000; *Nat Med* **6**: 564-657; Nitsch *et al.* 2000, *Lancet* **356**: 827-828). The latter finding suggests a two edged role for TRAIL in neuroinflammation by promoting both anti-inflammatory and destructive properties. However, given the immediate effects of IFN-β on the integrity of the blood-brain barrier, the IFN-β-induced TRAIL up-regulation in the treatment of MS more likely comprises immunoregulatory mechanisms in the periphery than direct effect on the CNS (Stone *et al.* 1995, *Ann Neurol* **37**: 611-619).

Up till now it has not been possible to predict a patient's susceptibility to IFN-β treatment with a high level of confidence. Given that the IFN-β treatment is extremely expensive and places an immense financial burden on the health system, it would be desirable to be able to make predictions about the outcome of an IFN-β therapy to a patient. Moreover, for the individual patient it is highly desirable to have such a prediction in order to weigh up the probability of benefit on the one hand and side-effects that have to be expected on the other.

Accordingly, it has been an object of the present invention to be able to predict the chances of a successful IFN-β therapy in MS affected individuals.

This object is solved by a method as described in the appended claims.

A method to predict and/or monitor an individual's susceptibility to type-I Interferon (IFN) therapy used in the treatment of Multiple Sclerosis, characterized in that in a sample of mammalian origin TRAIL gene expression and/or soluble TRAIL protein level is determined, wherein
- TRAIL gene expression at least 2-fold elevated after 4 weeks of IFN therapy compared to the level prior to therapy is predictive of a positive therapeutic response, and/or
- soluble TRAIL protein level prior to IFN therapy above a limiting value of 350 ±20 pg/ml is predictive of a positive therapeutic response to IFN for at least 83.6% of the individuals.

A method to predict and/or monitor an individual's susceptibility to type-I Interferon (IFN) therapy used in the treatment of Multiple Sclerosis, characterized in that in a sample of mammalian origin TRAIL protein level is determined prior to IFN therapy and/or 12 months after initiation of IFN therapy, wherein
- soluble TRAIL protein level above a limiting value of 500 pg/ml is predictive of a positive response to IFN.

It is also solved by the use of the method according to the present invention for monitoring type-I IFN therapy used in the treatment of MS.

It is furthermore solved by the use of a kit or perforrning the method according to the present invention, comprising a nucleic acid probe and/or one or more primers specific for the TRAIL gene or a portion thereof and/or an antibody specific for the soluble TRAIL polypeptide or a portion thereof.

Preferably, the method of the present invention is used in an IFN-β therapy.

The method of the present invention is preferably used to determine TRAIL expression and/or TRAIL protein level prior to IFN therapy.

In said method, a sample is preferably obtained from a mammalian, and more preferably is obtained from a human.

In a preferred embodiment, the sample is selected from the group comprising a bodily fluid, a fraction thereof, tissue extract, and cell extract.

In a more preferred embodiment, the bodily fluid is selected from the group comprising whole blood, blood serum, plasma, cerebrospinal fluid, synovial fluid, ascites exsudate, inflammatory exsudate, and urine.

In a particular preferred embodiment of the method according to the present invention,
(a) TRAIL gene expression is determined by detecting and/or measuring the amount and/or concentration of TRAIL nucleic acids encoding the TRAIL polypeptide or parts thereof, and
(b) TRAIL protein level is determined by detecting and/or measuring the amount and/or concentration of soluble TRAIL polypeptide or parts thereof.

In one embodiment of the method according to the present invention, soluble TRAIL protein level determination is performed by measuring the amount and/or concentration of soluble TRAIL (sTRAIL) protein in a liquid sample.

The measurement of the amount and/or concentration of soluble TRAIL polypeptide is preferably done by an immunoassay using an anti-TRAIL antibody.

The immunoassay preferably is an enzyme-linked immunosorbent assay (ELISA) but also may be, for example, a radioimmunoassay (RIA) or a blotting technique such as Western blot.

The amount and/or concentration of soluble TRAIL polypeptide determined prior to IFN therapy is compared to a limiting value discriminating positive responders and non-responders, which limiting value is 350 ± 20 pg/ml.

In another embodiment of the present invention, the amount and/or concentration of TRAIL nucleic acid is determined by preferably measuring the amount and/or concentration of TRAIL mRNA in a sample of tissue or cell extract.

In a preferred embodiment, said sample comprises an extract selected from the group comprising an extract of blood cells, peripheral immune cells, neuronal tissue and muscle tissue, of which an extract of peripheral blood mononuclear cells (PBMC) is particularly preferred.

Measurement of the amount and/or concentration of TRAIL mRNA is preferably done with the aid of the polymerase chain reaction (PCR) using one or more TRAIL specific probes.

Alternatively, other techniques for determining nucleic acids such as Northern blot may be used. Furthermore, the amount and/or concentration of TRAIL mRNA may be determined in solid tissue or cell specimens by, for example, *in situ* hybridization using TRAIL specific probes.

The amount and/or concentration of TRAIL mRNA is compared to a limiting value discriminating positive responders and non-responders. Said limiting value is at least twofold compared to the baseline.

The amount and/or concentration of TRAIL nucleic acid and/or polypeptide is preferably correlated with the amount and/or concentration of a nucleic acid and/or polypeptide, respectively, of one or more biological response markers of IFN-β therapy, such as MxA protein or any other IFN-inducible protein.

As used herein, an individual suffering from manifested MS is diagnosed according to generally accepted criteria (Poser *et al.* 1983, *Ann Neurol* **13**: 227-231; McDonald *et al.* 2001, *Ann Neurol* **50**: 121-127). Disability status (as defined by the Expanded Disability Status Scale, EDSS; Kurtzke 1983, *Neurology* **22:** 1444-1452) was assessed by clinical parameters.

As used herein, "positive responders" are defined as patients who experienced no further relapses and no deterioration in the EDSS during IFN-β treatment, whereas patients who continue to have one or more relapses are defined as "non-responders". In another aspect, positive responders and non-responders to IFN-β treatment are defined by paraclinical activity markers, e.g. magnetic resonance imaging (MRI).

The method of the present invention shall be understood as a method wherein determination of TRAIL expression and/or TRAIL protein level is carried out extracorporally.

The term "determination" as used herein may mean both qualitatively detecting and quantifying.

As used herein; an anti-TRAIL antibody is an antibody directed against soluble TRAIL polypeptide or parts thereof and may comprise polyclonal antiserum, monoclonal antibodies, diabodies and chimera.

In summary, TRAIL is disclosed herein as the first biological response marker of IFN-β therapy in MS that has been identified. It is demonstrated that early and sustained TRAIL gene induction is a marker of IFN-β therapy response by linking its gene and protein expression profile with clinical disease markers in long-term treated MS patients. More detailed, non-responders to IFN-β therapy can be distinguished from responsive individuals by their *in vivo* profile of TRAIL gene and protein expression. Furthermore, elevated sTRAIL protein levels in patient sera allowed to predict treatment response even before therapy was initiated. Using an ELISA for determining sTRAIL serum concentration, a limiting value of about 350 ±20 pg/ml was estimated. Thus, concentrations above that limiting value indicate a prediction of positive response to IFN-β therapy, whereas lower concentrations point to a response failure.

The invention shall now be further described by the following examples with respect to the attached figures. All examples are provided by way of example only, without any intended limitation of the scope of the invention.

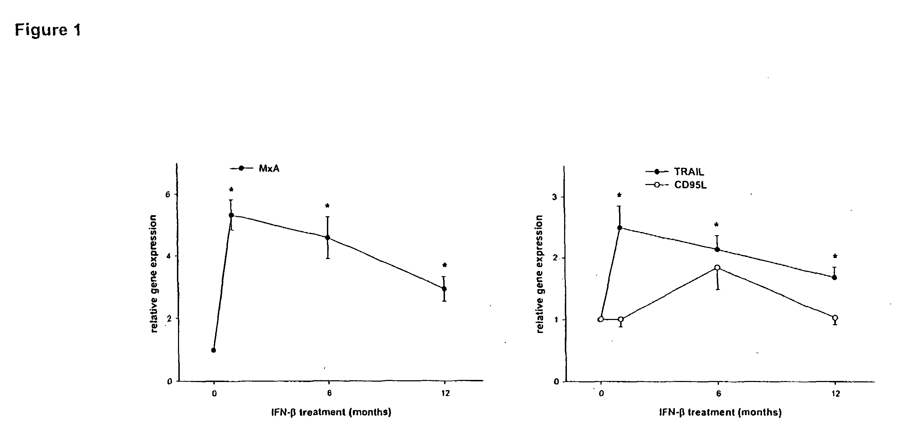

Prior to outlining the examples, reference is made to the figures, wherein
**Figure 1** shows the spontaneous gene expression of MxA, TRAIL and CD95L-in 62 MS patients during IFN-β-1a therapy. Samples of peripheral blood mononuclear cells (PBMC) were obtained before the beginning of IFN-β-1a therapy and at weeks 4, 26 and 52 under therapy. Total RNA was extracted and relative mRNA levels were quantified by realtime (rt)-PCR. Gene expression levels were normalized to expression of hypoxanthine phosphoryltransferase (HPRT). Induction of MxA protein gene expression was measured as a biological response marker to IFN-β therapy *in vivo*. Changes are expressed relative to gene expression levels before treatment. Data are presented as mean ± SEM. Statistical significance of changes after 4, 26 and 52 weeks of treatment compared to baseline was tested by Wilcoxon's signed-rank test-: *p < 0.05.

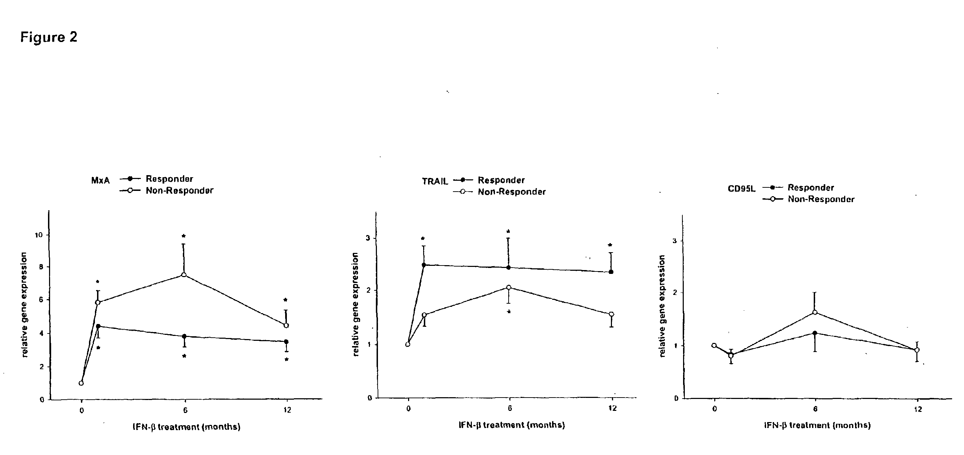

**Figure 2** shows the differential induction of TRAIL gene expression in IFN-β responders (n=20) and non-responders (n=19). In contrast to non-responders, IFN-β-1a therapy resulted in early and sustained up-regulation of TRAIL gene expression in MS patients with clinical treatment responser. Up-regulation of MxA gene expression demonstrates the *in vivo* biological effectiveness of IFN-β-1a in both patient groups. CD95L was not significantly regulated by IFN-β therapy in any patient subset. *p < 0.05.

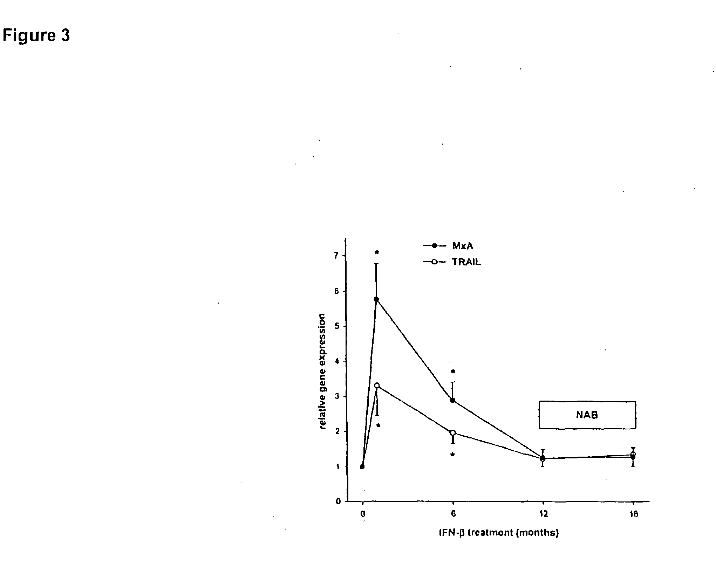

**Figure 3** shows that the up-regulation of TRAIL gene expression during IFN-β treatment is abrogated in the presence of neutralizing antibodies (NAB). Spontaneous gene_ expression of MxA and TRAIL was measured in a third subset of patients (n=23) who developed NAB to IFN-β-1a after 1 year of treatment. Presence of NAB was confirmed after 18 months. Initial up-regulation of MxA and TRAIL gene expression was abrogated in the presence of NAB. *p <0.05.

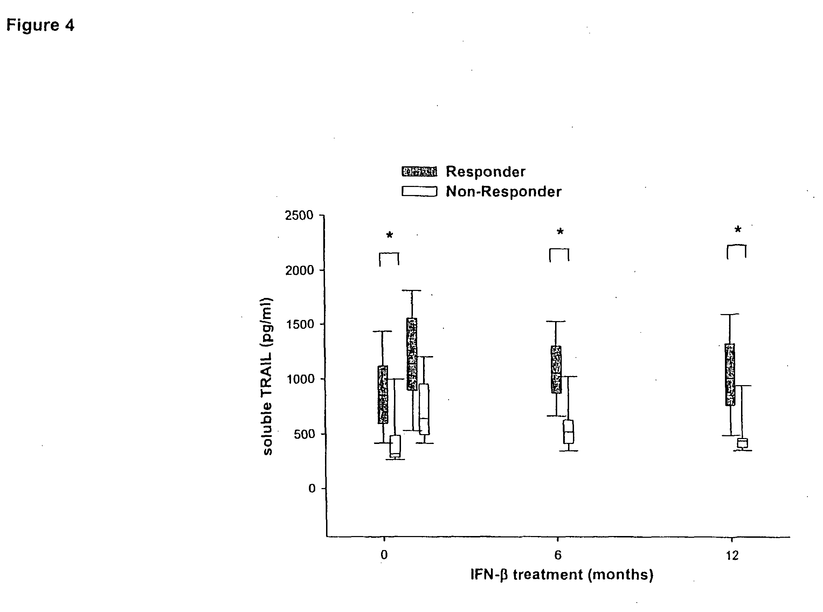

**Figure 4** shows that elevated sTRAIL protein levels are correlated with IFN-β responsiveness in MS patients. Soluble TRAIL protein levels were measured in serum of IFN-β responders (n=12) and non-responders (n=11) before the beginning of IFN-β-1a therapy and at weeks 4, 26 and 52 under therapy. Box-whisker plots depict the median level for each group (horizontal line in the box), the 25^{th} and 75^{th} percentiles (upper and lower edges of box), and the 10^{th} and 90^{th} percentiles (lines extending above and below the box). Statistical significance of differences between the patient group was tested by the Mann-Whitney-U test. *p < 0.05.

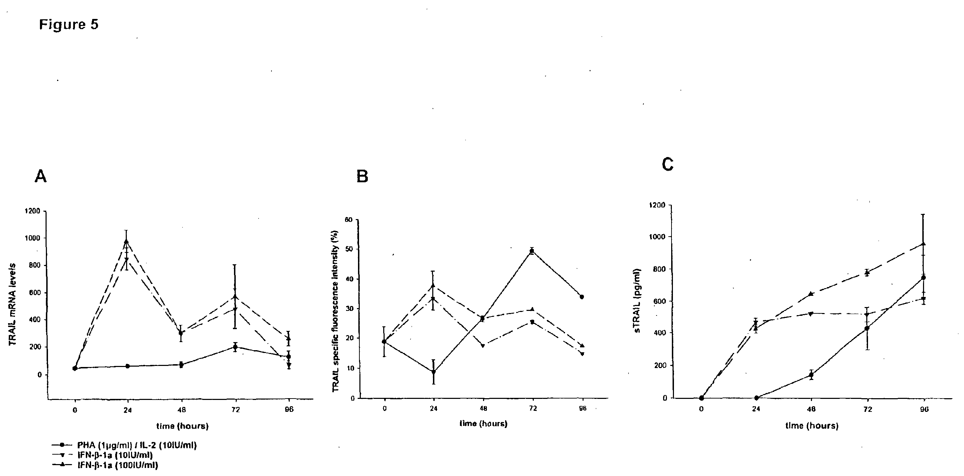

**Figure 5** shows the regulation of TRAIL gene transcription and protein expression by IFN-β. PBMC (10⁶ per well) were cultured for the indicated time periods in 48 well plates in a final volume of 1 ml. Cells were incubated with IFN-β-1a (10 and 100 IU/ml) of PHA (1µg/ml) and Interleukin-2 (IL-2) (10 IU/ml). TRAIL mRNA was quantified by rtPCR. Gene expression levels were normalized to 18S rRNA (A). Cell surface expression was detected by flow cytometry. Cells were stained with an anti-TRAIL monoclonal antibody, followed by phycoerythrin (PE)-labeled goat anti-mouse monoclonal antibody (B). Concentration of sTRAIL protein in culture supemates was determined by ELISA (C). Results obtained in 2 healthy individuals are shown (mean ± SEM).

### Examples

The following examples are intended to illustrate and to substantiate the present invention. In order to study an involvement of TRAIL in IFN-β therapy, patients with manifested MS were recruited. The study was approved by the local ethical committee, and informed consent was obtained from each individual.

All MS patients had clinically definite, relapsing-remitting MS. Healthy volunteers were selected from personnel of the Neuroimmunology lab. MS patients (n=62) studied longitudinally participated in the MASTER (MS Antibody Status and Therapy Evaluation with Rebif®) study, a clinical trial of IFN-β-1a. Eligible subjects for that study were patients at ages between 18 and 55 years who had relapsing-remitting MS for more than two years, with at least two relapses during the past two years, without any immunomodulatory treatment six months prior to the study, and without exacerbation 4 weeks prior to treatment initiation (Lünemann *et al.* 2001, *Neurology* **57**: 1132-1134). All patients were treated with 44 µg IFN-β-1a (Rebif®, Serono, Unterschleißheim, Germany) by subcutaneous injection once weekly. This dose has been shown to significantly decrease the T2 activity and burden of disease as measured by MRI and represents twice the concentration of IFN-β-1a- that has been demonstrated to delay the conversation to definite MS in patients after a first episode of neurological dysfunction (The Once Weekly Interferon for MS Study Group 1999, *Neurology* **53**: 679-686;-Comi *et al.* 2001, *Lancet* **357**; 1576-1582). Furthermore, this dosage was found to be biologically effective as indicated by the clear and significant enhancement of MxA gene expression in PBMC after initiation of treatment (*cf* Fig. 1). Clinical data on disability status (as defined by the EDSS) and relapses were obtained every time of blood drawing 3 days after IFN-β-1a application (Kurtzke 1983, *Neurology* **33**: 1444-1452). A clinical relapse was defined as significant worsening for preexisting symptoms or appearance of new neurological deficits in the absence of fever and lasting for more than 24 hours. Patients who experienced no further relapses and no deterioration in the EDSS during IFN-β treatment were defined as positive responders, whereas patients who continued to have one or more relapses were defined as non-responders. In some of these patients, clinical relapses were treated with a 3-day course of i.v. methylprednisolone at a dose of 1 g/day. However, all blood samples were obtained before treatment. For the purpose of this study, positive responders (n=20) and non-responders (n=19) were randomly selected (*cf* Table 1). In addition, patients (n=23) who developed neutralizing antibodies (NAB) to Rebif® as determined by means of an MxA induction assay were included. The MxA concentration induced by Rebif® was determined as described (Kracke *et al*. 2000, *Neurology* **54**: 193-199). Results are given as. Interferon Neutralizing Units (INU), i.e.. the titer of serum that neutralizes 10 U/ml Rebif® activity to an apparent 1 U/ml activity as determined by a standard curve. Within this group, 17 patients fulfilled the clinical criteria for treatment response. Venous blood samples were collected prior to IFN-β treatment (baseline = week 0) as well as at weeks 4, 26 and 52 under therapy (each time 3 days after IFN-β application). Patients who developed NAB during the treatment course were followed up until week 78.

### Example 1: Induction of TRAIL gene expression upon systemic IFN-β treatment

First, it was examined whether systemic IFN-β treatment of MS patients induces TRAIL gene expression *in vivo.* For this purpose, PBMC from MS patients (n=62) before and during IFN-β-1a therapy were investigated.

Samples of peripheral blood was obtained before the beginning of IFN-β-1a therapy and at weeks 4, 26 and 52 under therapy. PBMC were isolated from fresh blood by Ficoll density gradient centrifugation (Bio-Whittaker, Walkersville, MD) and cryopreserved in liquid nitrogen until analysis was done.

Total RNA was extracted and relative mRNA levels were quantified by realtime (rt)-PCR. In addition, the closely related TNF family member CD95L (also referred to as FasL) was quantified. In parallel, MxA protein gene expression was measured as an *in vivo* response marker to IFN-β-1a therapy (Wandinger *et al.* 2001, *Ann Neurol* **50**: 349-357).

Isolation of total RNA from cells was carried out using the RNeasy Kit (Qiagen, Santa Clarita, CA), reverse transcribed to cDNA with random hexamers using the TaqMan® Reverse Transcription Reagents as per manufacturer's instructions (Perkin Elmer, Foster City, CA). Quantitative rtPCR was performed on an ABI Prism® 7700 Sequence Detection System (Perkin Elmer) (Wandinger *et al.* 2001, *Ann Neurol* **50:** 349-357). Amplification of hypoxanthine phosphoryltransferase (HPRT) and 18S rRNA for stimulated conditions was used for sample normalization. The amplification protocol followed the suggestions of the TaqMan® Gold RT-PCR kit. For detection of TRAIL, CD95L, MxA and HPRT transcripts, oligonucleotides were used at final concentrations of 200 nM for forward and reverse primer and 100 nM for the fluorogenic probe as follows:

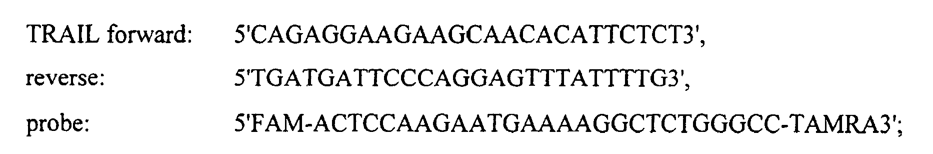

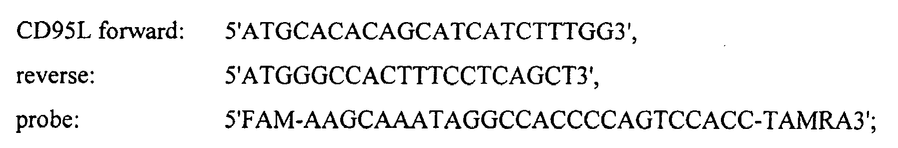

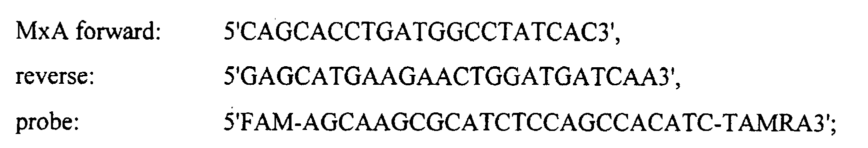

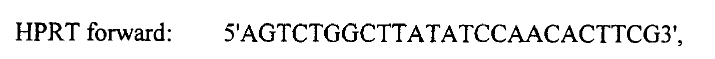

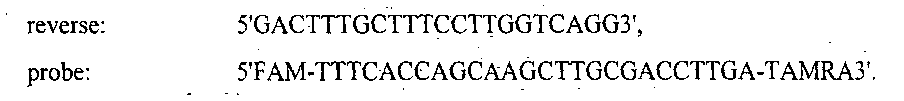

Quantification of gene expression relative to HPRT or 18S rRNA was calculated by the protocol's ΔΔC_{T} method. 18S rRNA was amplified using TaqMan® Ribosomal RNA Control Reagents (Perkin Elmer). Changes in gene expression of twofold or more were considered valid for further statistical analysis.

Statistical analysis was performed , using SPSS software (SPSS Software GmbH, . Munich, Germany). The Mann-Whitney-U test was used to compare data between patient groups. For analysis of dependent variables within groups, Wilcoxon's matched pair signed-rank test was performed. Data obtained from experiments outlined below were evaluated using the same statistical approach.

In Fig. 1 it is shown that, compared to baseline, spontaneous gene expression of both MxA and TRAIL was significantly up-regulated in PBMC of MS patients after 3 weeks of treatment and remained elevated for a period of 1 year. In contrast, CD95L did not show any significant regulation during IFN-β therapy at each time-point tested.

### Example 2: TRAIL gene expression in IFN-β responders and non-responders

In order to examine the functional relevance of TRAIL induction for the mechanism of action of IFN-β, patients were characterized with regard to their clinical response status. Patients, negative for NAB who experienced no further relapses and no deterioration in the EDSS during IFN-β treatment were defined as positive drug responders, whereas patients who continued to have one or more relapses were defined as non-responders.

As illustrated in Fig. 2, IFN-β therapy resulted in early and sustained up-regulation of TRAIL gene expression in MS patients with clinical response. Conversely, TRAIL gene expression was only transiently induced after 6 months of treatment in drug non-responders. The biological response marker MxA was significantly up-regulated in both groups (Fig. 2A), indicating the *in vivo* biological effectiveness of IFN-β-1a in both patient groups. As outlined in Fig. 2A, non-responders showed even higher levels of MxA gene expression in response to systemic IFN-β treatment at each time point investigated. As for the whole group, CD95L was not significantly regulated by IFN-β therapy in the patient subsets. No differences in baseline expression levels of the genes investigated were observed between responders and non-responders (data not shown).

### Example 3: TRAIL gene expression in patients developing neutralizing antibodies

Given the parallel up-regulation of MxA and TRAIL expression by IFN-β *in vivo,* it was investigated whether the occurrence of NAB in the course of IFN-β treatment influences TRAIL gene expression as reported for MxA (Deisenhammer *et al.* 1999, *Neurology* **52**: 1239-1243). Therefore, spontaneous gene expression was analyzed in a third subset of patients who developed NAB after 1 year (n=23). Presence of NAB was confirmed after 18 months (*cf* Table 1).

Fig. 3 demonstrates that in these subjects, bioavailability of IFN-β as measured by MxA gene induction was completely inhibited in the presence of NAB. In parallel, initial up-regulation of TRAIL gene expression by IFN-β was abrogated, indicating a direct regulation of TRAIL transcription by IFN-β. The expression of CD95L did not show significant variations in the presence of NAB (data not shown).

Interestingly, the majority of these patients remained in the clinical response status in the study time. However, this clinical observation is in keeping with the results of the pivotal trial of IFN-β-1b and the extension of the PRISMS study of IFN-β-1a, where development of NAB was finally associated with a delayed loss of efficacy in an observation period between 18 and 24 months and after 24 months, respectively (The IFNB Multiple Sclerosis Group and the University of British Columbia MS/MRI Analysis Group 1996, *Neurology* **47:** 889-894; The PRISMS Study Group and The University of British Columbia MS/MRI Analysis Group. PRISMS-4, 2001, *Neurology* **56:** 1628-1636).

### Example 4: Protein expression of TRAIL in IFN-β responders and non-responders

To address the question whether IFN-β non-responders differed from responders also with regard to TRAIL protein expression, serum concentrations of sTRAIL protein before and during IFN-β-1a therapy was measured.

Serum of NAB negative drug responders (n=12) and non-responders (n=11), from whom serum was available for investigation, was stored at -20°C until the assay were performed. Soluble TRAIL protein was quantified using a sandwich ELISA with a detection limit of 65 pg/ml according to the manufacturer's protocol (Trinova Biochem, Gießen, Germany).

As demonstrated in Fig. 4, responders showed significantly higher sTRAIL baseline levels already prior- to beginning of treatment, indicating underlying differences in the post-transcriptional regulation of TRAIL expression. Levels of sTRAIL above 350 pg/ml were definitely associated with response to IFN-β therapy. Thus, levels of sTRAIL above or below 350 pg/ml prior to therapy predicted the clinical outcome in 92% of the positive responders and 73% of the non-responders, respectively. For the total group of patients, this implied a correct prediction rate of 83,6%. Compared to baseline levels, sTRAIL protein accumulation in the serum was significantly enhanced after 4 weeks of IFN-β treatment in responders and non-responders. In long-term treated patients, sTRAIL protein levels remained significantly up-regulated in positive responders compared to non-responders after 6 and 12 months.

In addition to sTRAIL in blood serum, membrane bound TRAIL protein expressed on PBMC was measured using flow cytometry. PBMC from NAB negative responders and non-responders before and during IFN-β-1a therapy were prepared and 2x10⁵ PBMC per sample were incubated with an anti-TRAIL monoclonal, antibody (cat. no. 804-322-C100, Alexis Corporation, San Diego, CA) or control mouse IgG1 (cat. no. X0931, DAKO, Glostrup, Denmark) at a concentration of 1:100 for 30 min at 4°C. Washed cells were incubated with a phycoerythrin (PE)-labeled secondary goat-anti-mouse antibody (cat. no. R0480, DAKO) at a concentration of 1:20 for 30 min. After washing with phosphate buffered saline (PBS), cells were analyzed on a FACScan flow cytometer and data were processed using the Cellquest software (Becton-Dickinson, Mountain View, USA). Values are expressed as specific fluorescence intensity, calculated by histogram statistics and defined by the percentage of fluorescence intensity with TRAIL antibody excluding the fluorescence intensity with the appropriate control antibody.

Significant changes in TRAIL surface expression on PBMC during IFN-β treatment in any of the patient subsets were not observed (data not shown). Since blood samples were collected from patients 3 days after IFN-β application (subcutaneous injection) throughout the study, the negative findings might be explained by the time course of membrane bound TRAIL expression.

### Example 5: TRAIL gene and protein expression in PBMC cell culture

PBMC were cultured in the presence of IFN-β-1a *in vitro* and the regulation of TRAIL gene transcription, cell surface expression and accumulation of soluble protein in culture supernates were investigated in comparison (*cf* Fig. 5).

Freshly thawed PBMC were resuspended in Iscove's modified Dulbecco's medium (Gibco, Grand Island, NY) supplemented with 2 mM L-glutamine, 50 mg/ml gentamycin and 100 U/ml penicillin/streptomycin (Whittaker Bioproducts, Gaithersburg, MD) and 5% human plasma. PBMC (10⁶ cells per well) were incubated in a humidified atmosphere of 5% CO₂ at 37°C. Where specified, phytohaemagglutinin (PHA) or cytokines were added to the cultures as follows: PHA 1 µg/ml (Sigma-Aldrich, Steinheim, Germany), recombinant human IFN-β-1a, 10 and 100 IU/ml (Rebif®, Serono, Unterschleissheim, Germany), recombinant human IL-2, 10 IU/ml (Proleukin, Eurocetus, Frankfurt, Germany).

As demonstrated in Fig. 5A, incubation of PBMC with IFN-β resulted in rapid, dose-dependent up-regulation of TRAIL mRNA levels after 24 hrs, followed by a second peak after 72 hrs. TRAIL-expression was likewise rapidly induced on the surface of PBMC after 24 hrs by IFN-β (Fig. 5B). However, expression of membrane bound TRAIL was only marginally enhanced compared to baseline levels after 72 hrs. Soluble TRAIL concentrations in culture supemates increased over 24 hrs and remained elevated for 96 hrs when stimulated with IFN-β at a concentration of 10 IU/ml (Fig. 5C). Incubation with IFN-β at a concentration of 1.00 IU/ml resulted in further increase of sTRAIL over 96 hrs. In contrast to IFN-β, stimulation of cells with PHA and IL-2 resulted in delayed TRAIL gene and protein expression (Fig. 5A-C). Consistent with previously reported findings, these data indicate that IFN-β directly regulates the transcriptional control of TRAIL expression (Kayagaki *et al.* 1999, *J Exp Med* **189**:1451-1460).

The features disclosed in the foregoing description, in the claims and/or in the accompanying drawings may, both separately and in any combination thereof, be material for realizing the invention in diverse forms thereof.

## Claims

1. A method to predict and/or monitor an individual's susceptibility to type-I Interferon (IFN) therapy used in the treatment of Multiple Sclerosis, **characterized in that** in a sample of mammalian origin TRAIL gene expression and/or soluble TRAIL protein level is determined, wherein
- TRAIL gene expression at least 2-fold elevated after 4 weeks of IFN therapy compared to the level prior to therapy is predictive of a positive therapeutic response, and/or
- soluble TRAIL protein level prior to IFN therapy above a limiting value of 350 ±20 pg/ml is predictive of a positive therapeutic response to IFN for at least 83.6% of the individuals.

2. A method to predict and/or monitor an individual's susceptibility to type-I Interferon (IFN) therapy used in the treatment of Multiple Sclerosis, **characterized in that** in a sample of mammalian origin TRAIL protein level is determined prior to IFN therapy and/or 12 months after initiation of IFN therapy, wherein
- soluble TRAIL protein level above a limiting value of 500 pg/ml is predictive of a positive response to IFN.

3. Method according to claim 2, wherein the protein level is measured after initiation of IFN therapy, preferably 6 months and/or 12 months after initiation of IFN therapy

4. Method according to one of the preceding claims, wherein the therapy used is an IFN-β therapy.

5. Method according to one of the preceding claims, wherein the mammalian is a human.

6. Method according claim 5, wherein the sample is selected from the group comprising a bodily fluid, a fraction thereof, tissue extract and, cell extract.

7. Method according to any of the preceding claims, wherein TRAIL gene expression is determined by detecting and/or measuring the amount and/or concentration of TRAIL nucleic acids encoding the TRAIL polypeptide.

8. Method according to claim 7, wherein the amount and/or concentration of TRAIL nucleic acids is determined by measuring the amount and/or concentration of TRAIL mRNA in a sample according to claims 1-5.

9. Method according to claim 8, wherein the sample is selected from the group comprising an extract of blood cells, peripheral immune cells, neuronal tissue and muscle tissue.

10. Method according to any of claims 8 and 9, wherein measurement of the amount and/or concentration of TRAIL mRNA is performed with the aid of the polymerase chain reaction using one or more TRAIL specific probes.

11. Method according to any of claims 1-6, wherein measurement of the amount and/or concentration of soluble TRAIL polypeptide is done by an immunoassay using an anti-TRAIL antibody.

12. Method according to any of the preceding claims, wherein the amount and/or concentration of TRAIL nucleic acids is correlated with the amount and/or concentration of nucleic acids of one or more member(s) of the group of type-I-IFN-inducible proteins, e.g. MxA protein, and/or the amount and/or concentration of soluble TRAIL polypeptide is correlated with the polypeptide amount and/or concentration of one or more member(s) of the group of type-I-IFN-inducible proteins, e.g. MxA protein.

13. Method according to any of the preceding claims, wherein the mammalian is negative for IFN-β-neutralizing antibodies (NAB-).

14. Use of a method according to any of claims 1-13, for monitoring IFN therapy used in the treatment of Multiple Sclerosis.

15. Use of a method according to any of claims 1-13, for predicting an individual's susceptibility to IFN therapy used in the treatment of Multiple Sclerosis.

16. Use of the determination of TRAIL gene expression and/or of the level of soluble TRAIL protein level in a sample of mammalian origin to predict an individual's susceptibility to Type I IFN therapy used in the treatment of Multiple Sclerosis.

17. Use according to claim 16, wherein TRAIL gene expression is determined prior to and after 4 weeks of therapy and/or the level of soluble TRAIL protein level is determined prior to IFN therapy and/or 12 months after initiation of IFN therapy.

18. Use of a kit, comprising a nucleic acid probe and/or primer(s) specific for the TRAIL gene and/or an antibody specific for the soluble TRAIL polypeptide for monitoring and/or for predicting an individuals susceptibility to Type I IFN therapy used in the treatment of Multiple Sclerosis.

## Patentansprüche

1. Verfahren zur Vorhersage und/oder der Überwachung der Empfindlichkeit eines Individuums auf Typ-I Interferon (IFN) Therapie, angewendet bei der Behandlung von Multipler Sklerose, **dadurch gekennzeichnet, dass** in einer Probe aus einem Säuger TRAIL Genexpression und/oder der Spiegel an löslichem TRAIL Protein bestimmt wird, wobei
- eine mindestens 2-fach erhöhte TRAIL Genexpression nach 4 Wochen IFN Therapie, verglichen mit dem Spiegel vor der Therapie für eine positive therapeutische Response prädiktiv ist, und/oder
- der Spiegel an löslichem TRAIL Protein vor der IFN Therapie oberhalb eines Grenzwertes von 350 ±20 pg/ml für eine positive therapeutische Response auf IFN für mindestens 83,6% der Individuen prädiktiv ist.

2. Verfahren zur Vorhersage und/oder der Überwachung der Empfindlichkeit eines Individuums auf Typ-I Interferon (IFN) Therapie, angewendet bei der Behandlung von Multipler Sklerose, **dadurch gekennzeichnet, dass** in einer Probe aus einem Säuger der TRAIL Proteinspiegel vor der IFN Therapie und/oder 12 Monate nach Beginn der IFN Therapie bestimmt wird, wobei
- der Spiegel an löslichem TRAIL Protein oberhalb eines Grenzwertes von 500 pg/ml für eine positive therapeutische Response auf IFN prädiktiv ist.

3. Verfahren nach Anspruch 2, wobei der Proteinspiegel nach Beginn der IFN Therapie, bevorzugterweise 6 Monate und/oder 12 Monate nach Beginn der IFN Therapie, gemessen wird.

4. Verfahren nach einem der voranstehenden Ansprüche, wobei die verwendete Therapie eine IFN-β Therapie ist.

5. Verfahren nach einem der voranstehenden Ansprüche, wobei der Säuger ein Mensch ist.

6. Verfahren nach Anspruch 5, wobei die Probe ausgewählt ist aus der Gruppe umfassend eine Körperflüssigkeit, eine Fraktion davon, Gewebeextrakt und Zellextrakt.

7. Verfahren nach einem der voranstehenden Ansprüche, wobei die TRAIL Genexpression durch Nachweisen und/oder Messen der Menge und/oder Konzentration von TRAIL Nukleinsäuren, die für das TRAIL Polypeptid kodieren, bestimmt wird.

8. Verfahren nach Anspruch 7, wobei die Menge und/oder Konzentration von TRAIL Nukleinsäuren durch Messen der Menge und/oder Konzentration von TRAIL mRNA in einer Probe nach den Ansprüchen 1-5 bestimmt wird.

9. Verfahren nach Anspruch 8, wobei die Probe ausgewählt ist aus der Gruppe umfassend einen Extrakt von Blutzellen, peripheren Immunzellen, neuronalem Gewebe und Muskelgewebe.

10. Verfahren nach einem der Ansprüche 8 und 9, wobei die Messung der Menge und/oder Konzentration von TRAIL mRNA mit der Hilfe der Polymerase Kettenreaktion unter der Verwendung von einer oder mehreren TRAIL spezifischen Sonden durchgeführt wird.

11. Verfahren nach einem der Ansprüche 1-6, wobei die Messung der Menge und/oder Konzentration von löslichem TRAIL Polypeptid durch einen Immuntest unter der Verwendung eines anti-TRAIL Antikörpers durchgeführt wird.

12. Verfahren nach einem der voranstehenden Ansprüche, wobei die Menge und/oder Konzentration von TRAIL Nukleinsäuren mit der Menge und/oder Konzentration von Nukleinsäuren von einem oder mehreren der Mitglieder aus der Gruppe von Typ-I-IFNinduzierbaren Proteinen, z.B. MxA Protein korreliert ist, und/oder die Menge und/oder Konzentration von löslichem TRAIL Polypeptid mit der Polypeptidmenge und/oder - konzentration von einem oder mehreren der Mitglieder aus der Gruppe von Typ-I-IFNinduzierbaren Proteinen, z.B. MxA Protein, korreliert ist.

13. Verfahren nach einem der voranstehenden Ansprüche, wobei der Säuger negativ für IFN-β-neutralisierende Antikörper (NAB-) ist.

14. Verwendung eines Verfahrens nach einem der Ansprüche 1-13 zur Überwachung der IFN Therapie, die bei der Behandlung von Multipler Sklerose angewendet wird.

15. Verwendung eines Verfahrens nach einem der Ansprüche 1-13, zur Vorhersage der Empfindlichkeit eines Individuums auf Typ-I Interferon (IFN) Therapie, die bei der Behandlung von Multipler Sklerose angewendet wird.

16. Verwendung der Bestimmung des Spiegels der TRAIL Genexpression und/oder des Spiegels an löslichem TRAIL Protein in einer Probe aus einem Säuger zur Vorhersage der Empfindlichkeit eines Individuums auf Typ-I Interferon (IFN) Therapie, die bei der Behandlung von Multipler Sklerose angewendet wird.

17. Verwendung nach Anspruch 16, wobei der Spiegel der TRAIL Genexpression vor und nach 4 Wochen an Therapie bestimmt wird and/oder der Spiegel an löslichem TRAIL Protein vor der IFN Therapie und/oder 12 Monate nach Beginn der IFN Therapie bestimmt wird.

18. Verwendung eines Kits, umfassend eine Nukleinsäuresonde und/oder Primer, die spezifisch für das TRAIL Gen sind, und/oder einen Antikörper, der spezifisch für das lösliche TRAIL ist, zur Überwachung und/oder der Vorhersage der Empfindlichkeit eines Individuums auf Typ-I Interferon (IFN) Therapie, angewendet bei der Behandlung von Multipler Sklerose.

## Revendications

1. Méthode pour prédire et/ou surveiller une susceptibilité de l'individu à la thérapie (IFN) de type I-Interferon utilisée dans le traitement de la Sclérose Multiple, **caractérisée en ce que** dans un échantillon d'origine mammifère est déterminée l'expression de gène de TRAIL et/ou le niveau de protéine soluble de TRAIL, où
- l'expression de gène de TRAIL au moins 2-fois élevé après 4 semaines de thérapie par IFN comparé au niveau avant de la thérapie est prédictif d'une réponse thérapeutique positive, et/ou
- le niveau de protéine soluble de TRAIL avant de la thérapie par IFN au-dessus d'une valeur limite de 350±20 pg/ml est prédictif d'une réponse thérapeutique positive à l'IFN pour au moins 83,6% d'entre les individus.

2. Méthode pour prédire et/ou surveiller une susceptibilité de l'individu à la thérapie (IFN) de type I-Interferon utilisée dans le traitement de la Sclérose Multiple, **caractérisée en ce que** dans un échantillon d'origine mammifère le niveau de protéine de TRAIL est déterminé avant de la thérapie par IFN et/ou 12 mois après l'initiation de la thérapie par IFN, où
- le niveau de protéine soluble de TRAIL au-dessus d'une valeur limite de 500 pg/ml est prédictif d'une réponse positive à l'IFN.

3. Méthode selon la revendication 2, où le niveau de protéine est mesuré après l'initiation de la thérapie par IFN, préférablement 6 mois et/ou 12 mois après l'initiation de la thérapie par IFN.

4. Méthode selon l'une des revendications antérieures, où la thérapie utilisée est une thérapie par IFN-β.

5. Méthode selon l'une des revendications antérieures, où le mammifère est un être humain.

6. Méthode selon la revendication 5, où l'échantillon est sélectionné d'un groupe comprenant un fluide corporel, une fraction de celui, extrait de tissu et, extrait de cellule.

7. Méthode selon l'une quelconque des revendications antérieures, où l'expression de gène de TRAIL est déterminée en détectant et/ou en mesurant la quantité et/ou la concentration en acides nucléiques de TRAIL codifiant le polypeptide de TRAIL.

8. Méthode selon la revendication 7, où la quantité et/ou la concentration en acides nucléiques de TRAIL est déterminée en mesurant la quantité et/ou la concentration de mRNA de TRAIL dans un échantillon selon les revendications 1-5.

9. Méthode selon la revendication 8, où l'échantillon est sélectionné d'un groupe comprenant un extrait de cellules de sang, cellules périphériques immunes, tissu neuronal et tissu de muscle.

10. Méthode selon l'une quelconque des revendications 8 et 9, où la mesure de la quantité et/ou de la concentration de mRNA de TRAIL est réalisée à l'aide de la réaction en chaîne de la polymérase en utilisant une ou plusieurs probes spécifiques de TRAIL.

11. Méthode selon l'une quelconque des revendications 1-6, où la mesure de la quantité et/ou de la concentration de polypeptide soluble de TRAIL est faite par un essai immun utilisant un anticorps anti-TRAIL.

12. Méthode selon l'une quelconque des revendications antérieures, où la quantité et/ou la concentration en acides nucléiques de TRAIL est corrélée avec la quantité et/ou la concentration en acides nucléiques de l'une ou plusieurs part(s) du groupe de protéines inductibles de type I-IFN, ex. la protéine MxA, et/ou la quantité et/ou la concentration de polypeptide soluble de TRAIL est corrélée avec la quantité de polypeptide et/ou la concentration de l'une ou plusieurs part(s) du groupe de protéines inductibles de type I-IFN, ex. la protéine MxA.

13. Méthode selon l'une quelconque des revendications antérieures, où le mammifère est négatif pour les anticorps de neutralisation (NAB-) de l'IFN-β.

14. Utilisation d'une méthode selon l'une quelconque des revendications 1-13, pour surveiller la thérapie par IFN utilisée dans le traitement de la Sclérose Multiple.

15. Utilisation d'une méthode selon l'une quelconque des revendications 1-13, pour prédire une susceptibilité de l'individu à la thérapie par IFN utilisée dans le traitement de la Sclérose Multiple.

16. Utilisation de la détermination de l'expression de gène de TRAIL et/où du niveau de protéine soluble de TRAIL dans un échantillon d'origine mammifère pour prédire une susceptibilité de l'individu à la thérapie de type I-IFN utilisée dans le traitement de la Sclérose Multiple.

17. Utilisation selon la revendication 16, où l'expression de gène de TRAIL est déterminée avant et après 4 semaines de thérapie et/ou le niveau de protéine de gène de TRAIL est déterminé avant de la thérapie par IFN et/ou 12 mois après l'initiation de la thérapie par IFN.

18. Utilisation d'un kit, comprenant une probe en acide nucléique et/ou amorce(s) spécifique(s) pour le gène de TRAIL et/ou un anticorps spécifique pour le polypeptide soluble de TRAIL pour surveiller et/ou pour prédire une susceptibilité de l'individu à la thérapie de type I-IFN utilisée dans le traitement de la Sclérose Multiple.


### SEQUENCE LISTING

<110> IPAL - Gesellschaft Patentverwertung Berlin mbH
   <120> Method for identifying type-I Interferon responsive MS patients by determining TRAIL expression
   <130> FB11395
   <140> EP 02 011964.0
   <141> 2002-05-02
   <160> 12
   <170> PatentIn version 3.1
<210> 1
   <211> 25
   <212> DNA
   <213> Homo sapiens
   <223> TRAIL forward PCR primer
   <400> 1
   cagaggaaga agcaacacat tctct 25
<210> 2
   <211> 25
   <212> DNA
   <213> Homo sapiens
   <223> TRAIL reverse PCR primer
   <400> 2
   tgatgattcc caggagttta ttttg 25
<210> 3
   <211> 27
   <212> DNA
   <213> Homo sapiens
   <223> TRAIL fluorogenic probe
   <400> 3
   actccaagaa tgaaaaggct ctgggcc 27
<210> 4
   <211> 22
   <212> DNA
   <213> Homo sapiens
   <223> CD95L forward PCR primer
   <400> 4
   atgcacacag catcatcttt gg 22
<210> 5
   <211> 20
   <212> DNA
   <213> Homo sapiens
   <223> CD95L reverse PCR primer
   <400> 5
   atgggccact ttcctcagct 20
<210> 6
   <211> 26
   <212> DNA
   <213> Homo sapiens
   <223> CD95L fluorogenic probe
   <400> 6
   aagcaaatag gccaccccag tccacc 26
<210> 7
   <211> 21
   <212> DNA
   <213> Homo sapiens
   <223> MxA forward PCR primer
   <400> 7
   cagcacctga tggcctatca c 21
<210> 8
   <211> 24
   <212> DNA
   <213> Homo sapiens
   <223> MxA reverse PCR primer
   <400> 8
   gagcatgaag aactggatga tcaa 24
<210> 9
   <211> 24
   <212> DNA
   <213> Homo sapiens
   <223> MxA probe fluorogenic probe
   <400> 9
   agcaagcgca tctccagcca catc 24
<210> 10
   <211> 25
   <212> DNA
   <213> Homo sapiens
   <223> HPRT forward PCR primer
   <400> 10
   agtctggctt atatccaaca cttcg 25
<210> 11
   <211> 22
   <212> DNA
   <213> Homo sapiens
   <223> HPRT reverse PCR primer
   <400> 11
   gactttgctt tccttggtca gg 22
<210> 12
   <211> 26
   <212> DNA
   <213> Homo sapiens
   <223> HPRT fluorogenic probe
   <400> 12
   tttcaccagc aagcttgcga ccttga 26